(19) Europäisches Patentamt
European Patent Office
Office européen des brevets

(11) Publication number: 0 142 192
A1

(12) # EUROPEAN PATENT APPLICATION

(21) Application number: 84201501.8

(22) Date of filing: 17.10.84

(51) Int. Cl.[4]: A 61 K 39/385
C 07 G 3/00, C 07 K 17/10
G 01 N 33/548

(30) Priority: 22.10.83 NL 8303646

(43) Date of publication of application: 22.05.85 Bulletin 85/21

(84) Designated Contracting States: BE CH DE FR GB IT LI LU NL SE

(71) Applicant: AKZO N.V.
Velperweg 76
NL-6824 BM Arnhem(NL)

(72) Inventor: Visser, Nicolaas
De Sering 21
NL-5831 RW Boxmeer(NL)

(72) Inventor: Greven, Hendrik Marie
Lage Lochemseweg 22
NL-7218 PA Almen(NL)

(74) Representative: Douma, Anno Dominicus et al,
Postbus 20
NL-5340 BH Oss(NL)

(54) **Preparation of immunogens consisting of antigenic determinates bound to glycoside-containing carriers.**

(57) The invention concerns a method for the preparation of immunogens comprising contacting antigenic determinants (such as oligosaccharides, oligonucleotides, polypeptides or haptens) bonded to hydrophobic residual groups with micelles of glycosides to effect the formation of mixed glycoside-hydrophobic residual group complexes to which antigenic determinants are bonded. The immunogens are useful as vaccines, and as immuno-chemical reagents. Prior to use the immunogens may be stabilized (e.g. by cross-linking of the antigenic determinants) and/or be protected against digestive degradation (e.g. by micro- or macro-encapsulation).

EP 0 142 192 A1

Croydon Printing Company Ltd.

# PREPARATION OF IMMUNOGENS CONSISTING OF ANTIGENIC DETERMINANTS BOUND TO GLYCOSIDE-CONTAINING CARRIERS.

The invention relates to the preparation of immunogens consisting of antigenic determinants bound to a carrier and vaccines which contain these immunogens.

Immunisation is employed extensively as a way to protect man and animals against infections by pathogenic organisms such as viruses, bacteria, parasites and the like. Active immunisation, which provides long-lasting protection, is preferred. Active immunisation is effected by introducing antigens of the pathogenic organism into the individual to be protected. Mostly this is done by inoculation with inactivated or attenuated strains of the pathogen. Inoculation with inactivated strains has the disadvantage that the immunizing power is often rather low due to the partial denaturation of the antigens which occurs during inactivation. Inoculation with attenuated strains of the pathogens entails the risk of a certain pathogenicity of these strains.

Hence, a safer and more efficient method of active immunization is desired. One possibility is, for example, inoculation with preparations which exclusively contain antigens instead of the complete pathogenic organisms. In this case, the achievement of very high purity, and in particular the complete removal of, for example, viral genetic material, is often difficult to attain. As more information continues to become available on the primary and spatial structure of the antigens, ways have been sought of using antigen fragments which are as small as possible, the so-called antigenic determinants, as immunogens for active immunization. An antigenic determinant is a part of the antigen which can act as a recognition site for a particular type of immunoglobulin directed against the antigen. When split off from the complete antigen, such small fragments are often easier to purify than the antigen itself, and are even small enough to permit their synthetic preparation.

However, it is found that inoculation with freely dissolved antigenic determinants results in much too low a level of immunization.

Significantly better immunization has been achieved by inoculation with an immunogen wherein antigenic determinants are bound to a carrier. Specifically, known carriers for this purpose are immunogenic macromolecules such as tetanus toxoid, key-hole limpet haemocyanin (KLH), multi-poly (DL-alanyl)-poly(L-lysine) and copolymers of D-aminoacids. The antigenic determinants, thus rendered more strongly immunogenic, are however still somewhat less immunogenic than, for example, the antigens in their natural form, from which the

antigenic determinants in question are derived. Moreover, the macromolecular carriers employed therein are less suitable for human administration and, for example, KLH is commercially of little interest because the material is so expensive.

There have now been found novel immunogens with antigenic determinants which appear to possess substantially better immunizing power than the immunogens wherein the carriers hitherto known are present. The carriers employed in the immunogens according to the invention are moreover non-pyrogenic and commercially more attractive than the known macromolecular carriers.

The immunogen according to the invention is prepared by contacting an antigenic determinant bonded to a hydrophobic residual group with a solution of glycosides having both a hydrophobic and a hydrophilic part, and present in an amount of at least the critical micelle concentration, under conditions wherein complexes are formed between the glycoside micelles and the hydrophobic residual groups, whereafter the resulting immunogenic particles are optionally stabilized and/or protected against digestive degradation.

Any glycoside having a hydrophobic part and a hydrophilic part can be used as the glycoside. Preferably, saponins are used, such as those described by Tschesche and Wulf, "Chemie und Biologie der Saponine" in Fortschritte der Chemie organischer Naturstoffe, edited by W. Herz, H. Grisebach and G.W. Kirby, Vol. 30 (1973) and in particular the highly polar saponins, and especially the polar triterpene-saponins such as the polar acidic bis-desmosides, for example saponin-extract from Quillaja bark, araloside

A, Chikusetsu-saponin IV, Calendula-glycoside C, Chikusetsu-saponin V, Achyranthes-saponin B, Calendula glycoside A, araloside B, araloside C, Putranjia-saponin III, Bersama saponiside, Putranjia-saponin IV, thrichoside A, trichoside B, saponaside A, trichoside C, gypsoside, nutanoside, dianthoside C, saponaside D, aescine from Aesculus hippocastonum or sapoalbin from Gypsophilla struthium, and in particular saponin extract from Quillaja saponaria Molina, preferably the DQ-extract prepared by the method of K. Dalsgaard ("Saponin Adjuvants", Bull.Off.int. Epiz 77 (7-8), 1289-1295 (1972)) and Quil A prepared by the method of K. Dalsgaard ("Saponin Adjuvants III", Archiv für die gesamte Virusforschung 44, 243-254 (1974)).

Mixtures of glycosides may also be used.

Antigenic determinants suitable for preparing immunogens according to the invented process are for example small polypeptides, small oligosaccharides, oligonucleotides, glycoprotein fragments and haptens.

With small polypeptides are meant here polypeptides comprising at least 4 up to about 40 amino acids. In general an antigenic determinant comprises not more than 4-8 amino acids. Sometimes, however, a somewhat larger number of amino acids is needed in order to ensure the specific spatial structure of the antigenic determinant; where appropriate, these extra amino acids can belong to the hydrophobic residual group. The polypeptides can for example be prepared synthetically on the basis of a known amino acid sequence, or can be obtained by proteolytic or chemical cleavage of larger polypeptides or proteins such as membrane proteins of

microorganisms or proteins of plant or animal origin or can be a polypeptide hormone or a fragment or analog thereof.

With small oligosaccharides in general are meant linear or branched chains of sugar units comprising at least 4 and up to about 20 sugar units, and more favorably between 6 and 10 sugar units. These oligosaccharides can be prepared synthetically or by chemical or enzymatic degradation of naturally occuring polysaccharides or glycoproteins.

With oligonucleotides are meant compounds consisting of at least 1 up to about 15 ribonucleotides or deoxyribonucleotides which are obtained synthetically or by enzymatic or chemical cleavage of polynucleotides such as RNA and DNA. Optionally these oligonucleotides may be doublestranded.

Glycoprotein fragments are prepared synthetically or by chemical and enzymatic cleavage of glycoproteins.

With haptens are meant molecules different from the polypeptides, oligosaccharides, oligonucleotides and glycoprotein fragments mentioned before and which are not suited for the induction of antibody formation unless bonded to a carrier. Examples of these are steroids and prostaglandines.

As hydrophobic residual group can be used any chemical group with hydrophobic character. Examples of these are hydrophobic polypeptides, or aliphatic chains, or fatty acid derivatives, or phospholipid derivatives.

The hydrophobic polypeptides can preferably comprise 4-50 amino acids and can in particularly consist of hydrophobic amino acid residues, such as valyl, phenylalanyl, tryptophyl, methionyl, leucyl and/or isoleucyl residues. Inter alia in the interests of appropriate solubility, for example in the course of the synthesis, it may be necessary to include a number of additional non-hydrophobic amino acids in the hydrophobic residual group. The hydrophobic polypeptides have the advantage that they are easy to couple synthetically to antigenic determinants, and specifically when using synthetically prepared polypeptide antigenic determinants it is an advantage that the synthesis of the hydrophobic residual group and the antigenic determinant can be carried out as one total operation.

Suitable aliphatic chains as hydrophobic residual groups are branched or straight hydrocarbon chains such as stearyl or palmityl.

Suitable also are groups derived from glycerol esters of branched or straight chain hydrocarbons, such as phosphatidyl derivatives.

The invention also includes the preparation of immunogens which comprise various types of antigenic determinants. These can be different antigenic determinants which are characteristic of one and the same pathogen, if desired for different serotypes of one and the same pathogen, or different antigenic determinants which are characteristic of different pathogens.

The preparation of immunogens according to the invention can be carried out in numerous possible ways.

Stated in general terms a solution of antigenic determinants to which hydrophobic radicals are coupled and to which, if desired for improvement of the solubility, a solubilizing agent, e.g. a detergent is added, is brought into contact with a glycoside solution which contains at least one glycoside which is composed of one hydrophobic and one hydrophilic part, in an amount at least equal to the critical micelle concentration, and at the same time or subsequently any solubilizing agent if present is removed.

Such a preparation can be carried out by dialysing or subjecting to ultrafiltration a solution of the antigenic determinants coupled to the hydrophobic residual groups, together with a solubilizing agent, in the presence of the said glycoside solution.

Specifically for the large-scale preparation of the immunogen according to the invention, ultrafiltration is a very suitable method.

However, it is also possible to prepare the immunogens by introducing a solution of the antigenic determinants, coupled to the hydrophobic residual groups, in the presence of solubilizing agent, onto a density gradient of, for example, sugar solutions or salt solutions, to which such an amount of glycoside containing a hydrophobic part and a hydrophilic part is added that the concentration is at least equal to the critical micelle concentration, and thereafter introducing the whole into a centrifugal force field, whereby the immunogen is separated out as a band.

Alternatively the antigenic determinant, coupled to the hydrophobic residual groups, may be sonicated in a solution also containing glycosides having a hydrophobic and a hydrophilic part in an amount of at least the critical micelle concentration, whereafter the complexes formed are isolated from the medium.

The immunogens according to the invention can be stabilized, if desired. This can be done, for example, by linking to themselves, or to one another, the antigenic determinants and/or the hydrophobic residual groups bonded thereto ("cross-linking").

The cross-linking can be established by any reagent possessing reactive groups and which are able to bond to at least two groups on the antigenic determinant and/or on the hydrophobic residual group. For the cross-linking on polypeptides generally use is made of glutaraldehyde or formaldehyde. Suitable cross-linking reagents are also homobifunctional reagents (such as bisamidates, bisuccinimidyl esters) or hetero bifunctional reagents (such as compounds which contain an azidophenyl group and an amidate or a succinimidyl or an activated fluorobenzene or a haloketone group).

The immunogen according to the invention is in particular suitable for vaccination. A vaccine containing the said immunogen can inter alia be used for immunization against a particular pathogen, for so-called priming (wherein the body is not immediately stimulated to form specific free antibodies but is effectively preconditioned so that, following infection or vaccination, for example with inactivated or attenuated pathogens,

a strong immunization reaction is brought about (see, inter alia, Emini et al. (1983) Nature 304, 699-703)), or for immunological sterilization or castration (whereby antibodies against substances vital to reproductive processes, such as certain hormones, are produced).

The invention therefore also includes vaccines which contain immunogens according to the invention. Such vaccines can be suitable for immunization or priming against a particular pathogen, with at least one type of antigenic determinant characteristic of the particular pathogen being present in the immunogen. A plurality of types of antigenic determinants of one and the same pathogen can also be present in the vaccine. At the same time it is possible for each immunogen to comprise a plurality of these antigenic determinants, or for each of the types of antigenic determinants to be present in separate immunogens. It is also possible, according to the invention, to compose vaccines for a plurality of pathogens, and in that case, once again, the respective antigenic determinants can be present in separate immunogens or conjointly in one immunogen.

The immunogens according to the invention can accordingly also be used in diagnostic tests, for example as a solid phase in an agglutination test, or as a marker phase in an immunochemical test; in the case of the last-mentioned use, the marker is, or can be, included in the immunogen.

To use the immunogen according to the invention for vaccination purposes, the immunogen must be brought into a suitable form for administration. For intramuscular or subcutaneous administration, the immunogens can, for example,

be mixed with physiological salt solution. For intranasal or intra-ocular administration, for example in the form of a spray, an aqueous solution will also preferably be used. For local (for example oral) administration it will in many cases be necessary for use a form which temporarily protects the immunogens (for example against saccharolytic enzymes in the oral cavity or against proteolytic enzymes in the stomach). Such protection may for example be effected by encapsulating the immunogens or by bringing them into a slow-release form or by using one or more aminoacids in the D-form on enzyme-sensitive sites of the antigenic determinants and/or hydrophobic residual groups.

By encapsulation is meant any method by which the pharmaceutical is (temporarily) protected against attack by certain desintegrating influences. Encapsulation may be established by covering any individual complex according to the invention with a protecting agent (micro capsules), or by encasing a multitude of complexes into a single protecting shield (macro capsules).

The encapsulating material may be semipermeable or may become (semi-)permeable if brought within the animal or human body or upon attaining certain sites within the animal or human body. In this way both encapsulation and sustained-release may be established by a single pharmaceutical preparation. For the encapsulation, therefore, generally a biodegradable material is used.

For the micro-encapsulation suitably the optionally cross-linked complexes according to the invention are dispersed into a solution of a biodegradable polymer, to which, optionally,

surfactants are added. Consequently the polymer is to build a capsule at the interface. The micro-capsules are separated and may be suspended into a physiologically acceptable medium. Alternatively the micro-capsules may be lyophylized.

Macro-encapsulation can be established by methods known in the art. Suitably the optionally cross-linked, complexes according to the invention are converted into an appropriate formulation compatible with oral (soft or hard) capsule material, such as gelatine, starch, etc. To this end the complexes to which are added for example mannitol, sucrose, glycerol, etc., may be dried or freeze-dried, whereafter the material can be granulated or suspended into for example a natural or synthetic gel and filled into capsules which have been enteric coated by, for example, formaldehyde, cellulose acetate phtalate, hydroxypropylmethyl cellulose phtalate or polyacrylic compounds. Alternatively enteric coated capsules are covered internally with a soft hydrophobic layer such as polysiloxane or synthetic or vegetable oils or waxes, prior to filling with the complexes according to the invention in aqueous suspended form.

The invention is further illustrated by reference to the examples below.

In these examples, the following abbreviations are used:

<u>for protective groups</u>:

| | |
|---|---|
| tBu | tertiary butyl |
| Bzl | benzyl |
| Z | benzyloxycarbonyl |

for solvents and reagents:

| | |
|---|---|
| DMF | dimethylformamide |
| DCC | dicyclohexylcarbodiimide |
| DCU | dicyclohexylurea |
| BuOH | butanol |
| TFA | trifluoroacetic acid |
| IAN | isoamyl nitrite |
| NEM | N-ethylmorpholine |

for aminoacid residues:

| | |
|---|---|
| Leu | leucyl |
| Gly | glycyl |
| pGlu | pyroglutamyl |
| His | histidyl |
| Trp | tryptophyl |
| Ser | seryl |
| Tyr - | tyrosyl |
| Arg | arginyl |
| Pro | prolyl |
| Val | valyl |
| Asn | asparaginyl |
| Asp | aspartyl |
| Gln | glutaminyl |
| Ala | alanyl |
| Thr | threonyl |
| Lys | lysyl |

for peptides:

| | |
|---|---|
| LH-RH | luteinising hormone releasing hormone. |

## Example 1

LH-RH immunogen

The product was prepared by twice in succession coupling the hexapeptide H-Leu-Leu-Leu-Leu-Leu-Gly-OtBu with the hydrochloride salt of acid LH-RH (pGlu-His-Trp-Ser-Tyr-Gly-Leu-Arg(HCl)-Pro-Gly-OH).

A. LH-RH-Leu-Leu-Leu-Leu-Leu-Gly-OH

The hydrochloride salt of acid LH-RH was dissolved in DMF and 2 equivalents of HOBt were then added. The mixture was then cooled to 0 °C and H-Leu-Leu-Leu-Leu-Leu-Gly-OtBu dissolved in DMF was added. This mixture was cooled further to -20 °C, after which 2 equivalents of DCC, dissolved in DMF, were added. The mixture was then stirred for 15 minutes at -15 °C, 2 hours at 0 °C and 12 hours at 40 °C. The reaction product was then cooled to -20 °C and the DCU which had precipitated was filtered off. The filtrate was evaporated and the residue dissolved in sec. BuOH:$CH_2Cl_2$ (2:3). This solution was washed 3 times with a bicarbonate solution and then 3 times with water, after which the organic layer was dried. Finally, ether was added to the solution obtained, whereupon the peptide precipitated. This peptide was deprotected in 90% TFA plus anisole under a stream of nitrogen gas, after which ether was added and the precipitate was filtered off, washed with ether and dried.

B. LH-RH[Leu-Leu-Leu-Leu-Leu-Gly]$_2$-OH

The product thus obtained was coupled to a second peptide Leu-Leu-Leu-Leu-Leu-Gly-OtBu in the manner described under A. and was then deprotected.

C. Immunogen of LH-RH

3 mg of the peptide described under B. were dissolved in 1 ml of a 2% strength Triton solution in 0.05 mol/l Tris HCl buffer of pH 7.2. This solution was mixed with 40 ml of an 0.2% strength solution of saponin Quil A in 0.05 mol/l of Tris HCl buffer of pH 7.2 and transferred into an ultrafiltration cell (Amicon No. 5122) provided with an ultrafiltration membrane having a cut-off of 1,000-2,000 D. Ultrafiltration took place in this cell with supply of the above-mentioned Tris HCl buffer, which was contained in a stainless steel supply vessel. When 0.5 litre of buffer had passed through the ultrafiltration membrane the solution in the ultrafiltration cell was concentrated, the ultrafiltration cell was then provided with an ultrafiltration membrane having a cut-off of 20,000 D and the solution was again ultrafiltered with 0.5 litre of the said buffer. Thereafter, the solution was concentrated until the final concentration of the immunogen formed was about 5 mg/ml. Under the electron microscope, spherical structures having a diameter of about 30 nm were observed.

Example 2

Mouth and foot disease immunogen

A. Z-Val-Pro-Asn-Leu-Arg(HCl)-Gly-Asp(OBzl)-Leu-Gln-Val-Leu-Ala-Gln-Lys(Z)-Val-Ala-Arg(HCl)-Thr-Leu-Pro-Leu-Leu-Leu-Leu-Leu-Gly-OH.

Z-Val-Pro-Asn-Leu-Arg(HCl)-Gly-Asp(OBzl)-Leu-Gln-Val-Leu-Ala-Gln-Lys(Z)-Val-Ala-Arg(HCl)-Thr-Leu-Pro-$N_2H_3$ was dissolved in DMF and the solution was cooled to 0 $^{o}$C. 2 Equivalents of HCl in DMF were added to this solution and the mixture was cooled to -20 $^{o}$C. 1 Equivalent of IAN was then added after

which the mixture was stirred for 15 minutes at -20 °C. Thereafter, 2 equivalents of NEM were added as well as 1 equivalent of the peptide H-Leu-Leu-Leu-Leu-Leu-Gly-OtBu dissolved in DMF; the pH was brought to 7.275 with NEM and the mixture was stirred for 3 days at 0 °C. The filtrate was then evaporated, the residue taken up in sec. BuOH:$CH_2Cl_2$ (2:3) and this solution washed successively 3 times with bicarbonate solution and 3 times with water and then dried. Ether was added to the solution and the precipitated peptide was filtered off. The C-terminal tBu group was split off with 90% TFA plus anisole under a stream of nitrogen gas. The reaction product was precipitated with ether, washed with ether and dried.

B. Z-Val-Pro-Asn-Leu-Arg(HCl)-Gly-Asp(OBzl)-Leu-Gln-Val-Leu-Val-Gln-Lys(Z)-Val-Ala-Arg(HCl)-Thr-Leu-Pro-[Leu-Leu-Leu-Leu-Leu-Gly]$_3$-OH.

This peptide was prepared by coupling the product obtained under A. twice in succession with Leu-Leu-Leu-Leu-Leu-Gly-OtBu in a corresponding manner to that described in Example 1A.

C. H-Val-Pro-Asn-Leu-Arg-Gly-Asp-Leu-Gln-Val-Leu-Val-Gln-Lys-Val-Ala-Arg-Thr-Leu-Pro-[Leu-Leu-Leu-Leu-Leu-Gly]$_3$-OH.

The protective Z and OBzl groups were split off by treatment with methanesulphonic acid and thioanisole in trifluoroacetic acid. The free peptide was isolated as the TFA salt by adding the reaction mixture dropwise to ether and filtering off, washing and drying the precipitate.

D. Immunogen of foot and mouth disease peptide.

4 mg of the peptide prepared under C. were dissolved in 0.05 mol/l Tris HCl buffer of pH 7.2 which contained 2% Triton X 100. An ultracentrifuge tube was filled with a 20-50% sucrose gradient containing 0.2% of Quil A. A layer of a 15% sucrose solution containing 1% of Triton was introduced over this, and the peptide solution described above was introduced over this.

The centrifuge tube was centrifuged for 40 hours in a rotor type No. SW 65 at 20 °C and 65,000 revolutions per minute (average 300,000xg). The contents of the tube was then separated into fractions and the fraction wherein the desired immunogen was present was detected by an immunoassay (ELISA), employing antiserum against foot and mouth disease virus serotype $O_1K$.

Example 3

Klebsiella serotype 11 immunogen

A. Klebsiella serotype 11 capsular octasaccharide (K11 OS).

An octasaccharide characteristic of the Klebsiella serotype 11 capsular polysaccharide (K11 PS) is prepared according to Zigterman et al. XIIth Internat.Carbohydrate Symp. at Utrecht, the Netherlands; Vonk Publishers, Ed. Vliegenthart et al., Zeist, 1984, p. 308. In summary 5 mg of K11 PS to which $5 \times 10^{10}$ pfu bacteriopage Ø 11 in 6 ml of PBS is added is incubated during 24 hours at 37 °C.

The octasaccharide subsequently is isolated and purified by gel filtration on Sephadex G25 (Pharmacia).

An octasaccharide of the formula I is obtained (K11 OS)

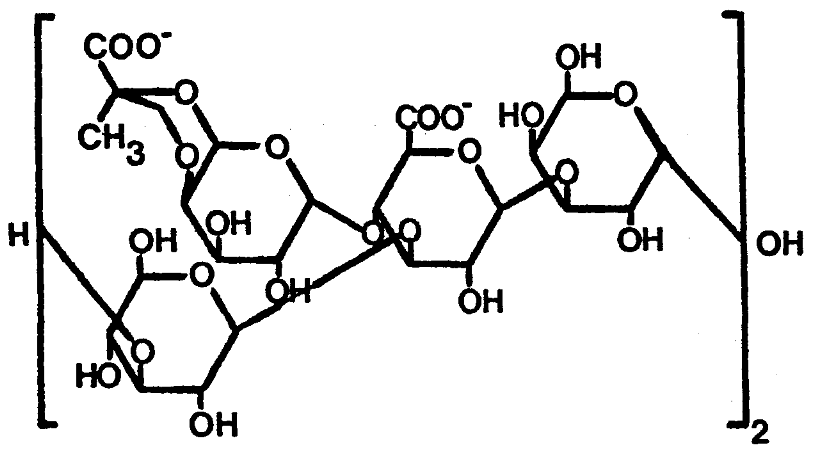

I

B. Coupling of stearylamine to K11 OS.

Stearylamine is coupled to the octasaccharide obtained under A. using the method described by Snippe et al. Infection and Immunity 42, 842-4 (1983). The coupling product is isolated and purified by gel filtration on Sephadex G25 (Pharmacia), and subsequently lyophilized.

C. Preparation of the immunogen from K11 OS-stearyl derivative.

The lyophilized product obtained under B. is dissolved in PBS to which 0.2% saponin Quil A is added. This solution is sonicated during 20 minutes at room temperature and the resulting complexes are dialyzed extensively against PBS.

## Example 4

β-endorphin-(6-17) immunogen

A solution of the dipalmitoyl-lysyl-derivative of β-endorphin-(6-17)

Palm-Lys-Thr-Ser-Glu-Lys-Ser-Gln-Thr-Pro-Leu-Val-
     |
     Palm

Thr-Leu-OH in trifluoroethanol is added to a solution of the saponin Quil A in methanol/water (3/1, v/v). The mixture is evaporated to dryness, and the residue is subsequently dispersed in 0.05 M sodium phosphate buffer, pH 7.4, 0.9% in NaCl, to give a mixture 0.5% (w/w) in Quil A and 1 mM in peptide. The dispersion is sonicated at 4 $^{o}C$ for 1 minute with a probe-type sonifier (Branson Sonifier 185, microtip, sonic power 40). The resulting particulate product is extensively dialysed at 4 $^{o}C$ against 0.05 M sodium phosphate buffer, 0.9% in NaCl, and subsequently freeze-dried.

## Example 5

Cross-linked immunogen

To the mouth and foot disease immunogen prepared according to Example 2, dissolved at 5 mg/ml in 0.1 M phosphate buffer pH 7.2, is added a freshly prepared solution of di(methyl-sulphonylethyl)-pimelidate,

$$CH_3-SC_2-CH_2-CH_2-O-\underset{\substack{\| \\ NH_2^{+}Cl^{-}}}{C}-(CH_2)_5-\underset{\substack{\| \\ NH_2^{+}Cl^{-}}}{C}-O-CH_2-CH_2-SO_2-CH_3,$$

to give a final concentration of 3 mM.

The mixture is kept at 4 $^{o}C$ for 4 hours, and is then dialysed for 20 hours against two changes of 20 mM phosphate buffer, pH 7.4, 140 mM in NaCl, and subsequently freeze-dried.

## Example 6

## Encapsulated mouth and foot disease immunogen

10 g of a 20% gelatin solution containing 0.1 g of the immunogen prepared according to Example 2 is extensively mixed with 25 g of liquid paraffin at 60 °C. The mixture is kept at 60 °C during 5 minutes and subsequently cooled to 5 °C and kept at that temperature during 90 minutes. Then 10 g of isopropanol is added and the particulate material is separated by suction filtration on a membrane filter and washed with isopropanol. Subsequently 1.5 ml of a 10% formaldehyde in isopropanol is added. The reaction is allowed at 7 °C for 24 hours. The thus hardened particles are then separated by filtration, washed with water and suspended in PBS.

CLAIMS

1. Process for the preparation of immunogens, characterized in that an antigenic determinant bonded to a hydrophobic residual group is contacted with a solution of glycosides having both a hydrophobic and a hydrophilic part, and present in an amount of at least the critical micel concentration, under conditions wherein complexes are formed between the glycoside micelles and the hydrophobic residual groups, whereafter the resulting immunogenic particles are optionally stabilized and/or protected against digestive degradation.

2. Process according to claim 1, characterized in that a solution of the antigenic determinant bonded to a hydrophobic residual group optionally containing a solubilizing agent is contacted with said solution of glycosides, whereafter the solubilizing agent, if present, is removed and the immunogen is isolated and/or purified.

3. Process according to claim 1 or 2, characterized in that the antigenic determinant is selected from the group consisting of small polypeptides, small oligosaccharides, haptens, polynucleotides, and glycoprotein fragments.
4. Process according to claim 1-3, characterized in that a mixture of two or more different antigenic determinants bonded to hydrophobic residual groups are used.
5. Process according to claim 1-4, characterized in that the immunogenic particles are stabilized by cross-linking of the antigenic determinants and/or hydrophobic residual groups.
6. Process according to claim 1-5, characterized in that the immunogenic particles are subsequently protected against digestive degradation by encapsulation.
7. Vaccines comprising an immunogen prepared according to any of the claims 1-6.
8. Vaccines comprising a plurality of different types of immunogens prepared according to any of the claims 1-6.
9. Immunochemical reagent comprising at least one immunogen prepared according to any of the claims 1-6.

European Patent Office

**0142192**
Application number

# EUROPEAN SEARCH REPORT

EP 84 20 1501

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 056 249 (NEW YORK BLOOD CENTER INC.)<br>* Claims 1,14; page 13, line 4 - page 15, line 4; page 15, lines 9-10, 15-35 *<br>--- | 1-9 | A 61 K 39/385<br>C 07 G 3/00<br>C 07 K 17/10<br>G 01 N 33/548 |
| A | GB-A-2 093 039 (NATIONAL RESEARCH DEVELOPMENT CORP.)<br>* Claims *<br>--- | 1-9 | |
| A | NATURE, Vol. 290, March 5, 1981, CHESHAM, BUCKS (GB). Page 51-54. J. SKELLY et al.:"Hepatitis B polypeptide vaccine preparation in micelle form".<br>* Page 51, right-hand column, lines 3-21 *<br>--- | 1-9 | TECHNICAL FIELDS SEARCHED (Int. Cl.4)<br>A 61 K |
| A | EP-A-0 058 021 (BEECHAM GROUP PLC)<br>* Claims 1,3,4,7; page 1, lines 16-20; page 2, lines 19-24 *<br>----- | 1-9 | |

The present search report has been drawn up for all claims

| Place of search | Date of completion of the search | Examiner |
|---|---|---|
| THE HAGUE | 04-02-1985 | RIJCKEBOSCH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82